(19) Europäisches Patentamt
European Patent Office
Office européen des brevets

(11) Numéro de publication: **0 060 767**
**B1**

# (12) FASCICULE DE BREVET EUROPEEN

(45) Date de publication du fascicule du brevet: **27.06.84**

(21) Numéro de dépôt: **82400405.5**

(22) Date de dépôt: **08.03.82**

(51) Int. Cl.³: **C 07 C 45/51,** C 07 C 49/203, C 07 C 49/647, C 07 C 49/607, C 07 C 49/587, C 07 C 175/00

## (54) Procédé de préparation de composés carbonylés delta-éthyléniques.

(30) Priorité: **09.03.81 FR 8104600**
**23.06.81 FR 8112300**

(43) Date de publication de la demande:
**22.09.82 Bulletin 82/38**

(45) Mention de la délivrance du brevet:
**27.06.84 Bulletin 84/26**

(84) Etats contractants désignés:
**AT BE CH DE FR GB IT LI LU NL SE**

(56) Documents cités:
**NL - C - 64 579**
**US - A - 3 929 893**

**JOURNAL OF THE AMERICAN CHEMICAL SOCIETY, volume 89, no. 14, 1967 A. VIOLA et al. "The vapor-phase thermolyses of 3-hydroxy-1,5-hexadienes II. Effects of methyl substitution", pages 3462-3470**
**HOUBEN WEYL, METHODEN DER ORGANISCHEN CHEMIE, volume VII/2a, Ketone, partie 1 GEORG THIEME VERLAG STUTTGART (DE)**

(73) Titulaire: **RHONE-POULENC SANTE, Les Miroirs 18 Avenue d'Alsace, F-92400 Courbevoie Cedex (FR)**

(72) Inventeur: **Bluthe, Norbert, 32 rue de la Baisse, F-69100 Villeurbanne (FR)**
Inventeur: **Gore, Jacques, 7 rue du Mont Cindre, F-69300 Caluire (FR)**
Inventeur: **Malacria, Max, 32 rue de la Baisse, F-69100 Villeurbanne (FR)**

(74) Mandataire: **Pilard, Jacques et al, RHONE-POULENC RECHERCHES Service Brevets Pharma 25, Quai Paul Doumer, F-92408 Courbevoie Cedex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 060 767 B1

BUNDESDRUCKEREI BERLIN

**Description**

La présente invention concerne un procédé de préparation de composés carbonylés $\delta$-éthyléniques de formule générale:

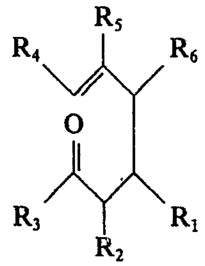

(I)

dans laquelle $R_1$, $R_2$, $R_4$, $R_5$ et $R_6$, identiques ou différents, représentent un atome d'hydrogène ou un radical hydrocarboné, $R_3$ représente un radical hydrocarboné, étant entendu que $R_1$ et $R_3$ peuvent représenter ensemble un radical triméthylène ou bien que $R_3$ et $R_4$ peuvent représenter ensemble un radical alcoylène $(-CH_2-)_n$ dont un ou plusieurs atomes de carbone peuvent être éventuellement substitués par un ou plusieurs radicaux alcoyles contenant 1 à 4 atomes de carbone et dans lequel n représente un nombre entier compris entre 3 et 20 inclusivement, par transposition d'un alcool diéthylénique de formule générale:

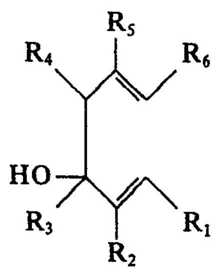

(II)

dans laquelle $R_1$, $R_2$, $R_3$, $R_4$, $R_5$ et $R_6$ sont définis comme précédemment.

Par radical hydrocarboné est entendu un radical acyclique contenent 1 à 20 atomes de carbone, dont la chaîne peut contenir une ou plusieurs doubles ou triples liaisons.

Les produits de formule générale (I) sont des intermédiaires particulièrement intéressants pour la préparation de produits ayant une activité biologique. Plus précisément, les produits de formule générale (I) trouvent leur application dans la synthèse de produits destinés à la pharmacie (vitamines A et E), l'agrochimie ou la parfumerie.

La transposition d'un alcool diéthylénique de formule générale (II) en un produit de formule générale (I) est une réaction connue appelée généralement transposition d'oxy-Cope ou de Cope énologène. La transposition d'oxy-Cope a fait l'objet de nombreux travaux mais compte tenu des conditions utilisées pour sa mise en oeuvre son intérêt pratique en a été très limité.

Par exemple, la thermolyse en phase vapeur selon le procédé décrit par A. Viola et coll., J. Amer. Chem. Soc., 89, 3462 (1967) est peu stéréosélective et elle conduit à des produits parasites de dégradation et de polymérisation dont l'origine est due aux hautes températures (voisines de 300°C) nécessaires au réarrangement.

Il a été montré, en particulier par Y. FUJITA et coll., Chem. Comm., 272 (1978) et Synthesis, 934 (1978), par M. L. ROUMESTANT et coll., Tetrahedron, 33, 1283 (1977) et par A. DOUTHEAU et coll., Tetrahedron, 36, 1953 (1980) que l'on augmentait le rendement et la stéréosélectivité en effectuant la réaction dans un solvant polaire aprotique, tel que la N-méthylpyrrolidone ou le diglyme, au reflux. Par ailleurs, D. A. EVANS et A. M. GOLOB, J. Amer. Chem. Soc., 97, 4765 (1975) ont montré que les alcoolates de potassium se transposaient plus facilement que les alcools eux-mêmes. Ainsi certains alcools de formule générale (II) traités par l'hydrure de potassium dans le tétrahydrofuranne au reflux conduisent aux produits carbonylés $\delta$-éthyléniques correspondants. Cependant ces conditions ne conviennent pas pour la transposition de composés dont la stabilité en milieu très basique est faible.

Il a maintenant été trouvé, et c'est ce qui fait l'objet de la présente invention, que la transposition d'oxy-Cope pouvait être réalisée une température comprise entre —40°C et 80°C, et de préférence entre —5 et 30°C, en opérant soit en présence d'une quantité pratiquement stoechiométrique d'un sel mercurique soit en présence d'une quantité catalytique d'un sel mercurique et d'une quantité pratiquement stoechiométrique d'un sel de lithium.

Lorsque la transposition est réalisée en présence d'une quantité pratiquement stoechiométrique d'un sel mercurique, on opère avantageusement en présence de chlorure mercurique ou d'acétate mercurique rendus suffisamment électrophiles par l'utilisation d'un solvant très polaire tel qu'un

mélange de tétrahydrofuranne et d'eau. Les meilleurs résultants sont cependant obtenus en utilisant le trifluoroacétate mercurique qui, tout en étant plus électrophile que le chlorure ou l'acétate mercurique, présente de plus l'avantage d'être soluble dans un certain nombre de solvants organiques tels que le chlorure de méthylène.

La cétone δ-éthylénique de formule générale (I) est généralement isolée après traitement du mélange réactionnel par un agent réducteur en milieu basique. De préférence, on utilise le borohydrure de sodium dans une solution aqueuse de soude.

Lorsque la transposition est réalisée en présence d'une quantité catalytique de sel mercurique et d'une quantité pratiquement stoechiométrique de sel de lithium, on opère avantageusement en présence de trifluoroacétate mercurique qui présente l'avantage d'être soluble dans des solvants organiques tels que le chlorure de méthylène ou le benzène.

Le sel mercurique est généralement utilisé à raison de 0,01 è 0,3 mole par mole d'alcool diéthylénique de formule (II) mis en oeuvre. De préférence, un rapport molaire voisin de 0,2 convient particulièrement bien.

Les sels de lithium qui permettent d'obtenir les meilleurs résultats sont choisis parmi le trifluoroacétate ou le trifluorométhanesulfonate. Généralement le sel de lithium est utilisé à raison de 1 mole d'alcool diéthylénique de formule (II).

La réaction est généralement complète après 1 à 30 heures à une température comprise entre −5 et 30°C.

La cétone δ-éthylénique de formule générale (I) est généralement isolée après traitement du mélange réactionnel par une solution aqueuse basique, telle qu'une solution saturée de bicarbonate de sodium.

Le sel mercurique utilisé comme catalyseur peut être éventuellement régénéré et mis en oeuvre pour la réalisation ultérieure de transposition d'oxy-Cope.

La cétone δ-éthylénique de formule générale (I) peut aussi être isolée après traitement du mélange réactionnel par un agent réducteur en milieu basique tel que le borohydrure de sodium dans une solution aqueuse de soude.

La cétone δ-éthylénique de formule générale (I) peut être purifiée par application de méthodes physiques telles que la destillation ou la chromatographie.

Les exemples suivants, donnés à titre non limitatif, montrent comment l'invention peut être mise en pratique.

## Exemple 1

On maintient à une température voisine de 20°C un mélange de 0,126 g ($10^{-3}$ mole) de diméthyl-3,5 hexadiène-1,5 ol-3 et de 0,270 g de chlorure mercurique dans 5 $cm^3$ d'un mélange de tétrahydrofuranne et d'eau (1—1 en volumes). Après 4 heures de réaction, on filtre le mélange réactionnel pour éliminer le mercure métallique qui s'est formé.

Le mélange réactionnel est extrait par 3 fois 25 $cm^3$ d'éther éthylique. Après séchage sur sulfate de sodium et évaporation du solvant sous pression réduite (20 mm de mercure, 2,7 kPa), on obtient 0,040 g de méthyl-6 heptène-6 one-2 dont les caractéristiques sont les suivantes:

- P. E. $_{101,3\ kPa}$ = 142—144°C
- Spectre infra-rouge à partir d'un film liquide: bandes caractéristiques à 3070, 1720, 1650 et 890 $cm^{-1}$
- Spectre de RMN ($CDCl_3$, δ en ppm, J en Hz): 1,65 (s, 3 H); 1,7 à 2,0 (mt, 4 H); 2,10 (s, 3 H); 2,30 (t; J = 7, 3 H); 4,65 (s élargi, 2 H).

Les caractéristiques de ce produit sont en accord avec celles qui ont été décrites par Viola et coll., J. Amer. Chem. Soc., 89, 3462 (1967).

Le rendement est de 32% par rapport à l'alcool mis en jeu.

## Exemple 2

On maintient à une température voisine de 20°C un mélange de 0,126 g ($10^{-3}$ mole) de diméthyl-3,5 hexadiène-1,5 ol-3 et de 0,427 g de trifluoroacétate mercurique dans 5 $cm^3$ d'un mélange de tétrahydrofuranne et d'eau (1—1 en volumes). Après 10 minutes de réaction, on filtre le mélange réactionnel pour éliminer le mercure métallique qui s'est formé.

Le mélange réactionnel est extrait par 3 fois 25 $cm^3$ d'éther éthylique. Après séchage sur sulfate de sodium et évaporation du solvant sous pression réduite (20 mm mercure; 2,7 kPa), on obtient 0,054 g de méthyl-6 heptène-6 one-2, dont les caractéristiques sont identiques à celles du produit obtenu à l'exemple 1, avec un rendement de 43%.

Exemple 3

On maintient à une température voisine de 20° C un mélange de 0,126 g ($10^{-3}$ mole) de diméthyl-3,5 hexadiène-1,5 ol-3 et de 0,427 g de trifluoroacétate de mercure dans 7,5 $cm^3$ de chlorure de méthylène. Après 10 minutes de réaction, on ajoute au mélange réactionnel une solution de 0,010 g de borohydrure de sodium dans 0,67 $cm^3$ de soude 3 N. Le mélange réactionnel est extrait par 3 fois 25 $cm^3$ de chlorure de méthylène. Après séchage sur sulfate de sodium et évaporation du solvant sous pression réduite (20 mm de mercure; 2,7 kPa), on obtient, avec rendement de 75%, 0,096 g de méthyl-6 heptène-6 one-2 dont les caractéristiques sont identiques à celles du produit de l'exemple 2.

Exemple 4

En opérent dans les conditions de l'exemple 3 mais à partir de 0,126 g ($10^{-3}$ mole) de diméthyl-3,4 hexadiène-1,5 ol-3 et de 0,427 g de trifluoroacétate mercurique dans 7,5 $cm^3$ de chlorure de méthylène, on obtient 0,078 g d'octène-6 one-2, contenant 80% de forme E, dont les caractéristiques sont les suivantes:

- Spectre infra-rouge à partir d'un film liquide: bandes caractéristiques à 3020, 1720 et 970 $cm^{-1}$
- Spectre de RMN ($CDCl_3$; $\delta$ en ppm; J en Hz): 1,65 (dd, 3 H, J = 5 et 2); 1,7 à 2,0 (mt, 4 H); 2,1 (s, 3 H); 2,35 (t, 2 H, J = 7); 5,25—5,50 (mt, 2 H).

Le rendement est de 62% par rapport à l'alcool mis en oeuvre.

Exemple 5

En opérant dans les conditions de l'exemple 3 mais à partir de 0,152 g ($10^{-3}$ mole) d'hydroxy-3 (méthyl-2 propène-2 yl)-3 cyclohexène et de 0,427 g de trifluoroacétate de mercure dans 7,5 $cm^3$ de chlorure de méthylène, on obtient 0,023 g de (méthyl-2 propène-2 yl)-3 cyclohexanone-1 dont les caractéristiques sont les suivantes:

- Spectre infra-rouge à partir d'un film liquide: bandes caractéristiques à 3080, 1720, 1690 et 895
- Spectre de RMN ($CDCl_3$; $\delta$ en ppm, J en Hz): 1,7 (s large, 3 H); 1,5—2,3 (massif, 11 H); 4,8 (massif, 2 H).

Le rendement est de 15% par rapport à l'alcool mis en oeuvre.

Exemple 6

En opérant dans les conditions décrites à l'exemple 3 mais à partir de 0,140 g ($10^{-3}$ mole) de diméthyl-4,5 heptadiène-2,6 ol-4 et de 0,427 g de trifluoroacétate de mercure dans 7,5 $cm^3$ de chlorure de méthylène, on obtient 0,070 g de méthyl-4 octène-6 one-2 contenant 80% de forme E, dont les caractéristiques sont les suivantes:

- Spectre infra-rouge à partir d'un film liquide: bandes caractéristiques à 3015, 1720 et 975 $cm^{-1}$
- Spectre de RMN ($CDCl_3$; $\delta$ en ppm; J en Hz): 0,97 (d, 3 H, J = 7); 1,65 (dd; 3 H; j = 5 et 2); 1,7—1,95 (mt, 3 H); 2,07 (s, 3 H); 2,3 (massif, 2 H); 5,2—5,5 (mt, 2 H).

Le rendement est de 50% par rapport à l'alcool mis en oeuvre.

Exemple 7

On maintient à une température voisine de — 5° C un mélange de 0,195 g ($10^{-3}$ mole) d'isopropényl-4 diméthyl-3,7 octadiène-1,6 ol-3 et de 0,475 g de trifluoracétate mercurique dans 10 $cm^3$ chlorure de méthylène. On laisse réagir pendant 10 minutes à cette température puis on laisse remonter la température au voisinage de 20° C. Après 1 heure 30, on ajoute au mélange réactionnel une solution de 0,010 g de borohydrure de sodium dans 7,3 $cm^3$ de soude 0,3 N. Le mélange réactionnel est extrait par 3 fois 10 $cm^3$ de chlorure de méthylène. Les extraits organiques sont séchés sur sulfate de magnésium. Après filtration et concentration du solvant sous pression réduite (20 mm de mercure; 2,7 kPa), on obtient, avec un rendement de 10%, 0,019 g de diméthyl-6,10 undécadièn-6,9 one-2 dont les caractéristiques sont les suivantes:

— Spectre infra-rouge à partir d'un film liquide: bande caractéristique à 1720 $cm^{-1}$
— Spectre de RMN ($CDCl_3$, $\delta$ en ppm, J en Hz): 1,5 à 1,8 (M, 11 H); 1,95 (t, 2 H); 2,07 (s, 3 H); 2,34 (t, J=7, 2 H); 2,60 (dd, 2 H); 4,8 à 5,3 (mt, 2 H)

## Exemple 8

On maintient à une température voisine de 20° C un mélange de 0,166 g ($10^{-3}$ mole) d'isopropényl-2 vinyl-1 cyclohexanol-1 et de 0,423 g de trifluoroacétate mercurique dans 10 $cm^3$ de chlorure de méthylène. Après 5 minutes de réaction, on ajoute au mélange réactionnel 0,010 g de borohydrure de sodium en solution dans 0,66 $cm^3$ de soude 3 N. Le mélange réactionnel est extrait par 3 fois 10 $cm^3$ de chlorure de méthylène. Les extraits organiques sont séchés sur sulfate de magnésium. Après filtration et concentration de solvant sous pression réduite (20 mm de mercure; 2,7 kg kPa) on obtient 0,024 g de méthyl-5 cyclodécen-5 one-1 dont les caractéristiques sont les suivantes:

— Spectre infra-rouge à partir d'un film liquide: bandes caractéristiques à 2980 et 1710 $cm^{-1}$
— Spectre de RMN ($CDCl_3$; $\delta$ en ppm, J en Hz): 1,47 (s, 3 H); 1,55 à 2,15 (M, 10 H); 2,15 à 2,63 (M, 4 H); 5,2 (t, j=7, 1 H)

## Exemple 9

On maintient à une température voisine de 20° C un mélange de 0,125 g ($5 \cdot 10^{-4}$ mole) de divinyl-1,2 cyclododécanol-1 et de 0,215 g de trifluoroacétate mercurique dans 4 $cm^3$ de chlorure de méthylène. Après 1 heure 30 de réaction, on ajoute au mélange réactionnel 0,005 g de borohydrure de sodium en solution dans 0,33 $cm^3$ de soude 3 N. Le mélange réactionnel est extrait par 3 fois 10 $cm^3$ de chlorure de méthylène. Les extraits organiques sont séchés sur sulfate de magnésium. Après filtration et concentration du solvant sous pression réduite (20 mm de mercure; 2,7 kPa), on obtient, avec un rendement de 70%, 0,087 g de cyclohexadecen-5 one-1.

## Exemple 10

On maintient à une température voisine de 20° C sous atmosphère inerte un mélange de 0,630 g [$5 \cdot 10^{-3}$ mole] de diméthyl-3,5 hexadiène-1,5 ol-3, de 0,600 g [$5 \cdot 10^{-3}$ mole] de trifluoracétate de lithium et de 0,427 g [$10^{-3}$ mole] de trifluoroacétate mercurique dans 70 $cm^3$ de chlorure de méthylène fraîchement destillé. Après 5 heures de réaction, le mélange réactionnel est lavé par par 3 fois 25 $cm^3$ d'une solution aqueuse saturée de bicarbonate de sodium. Après séchage de la phase organique sur sulfate de magnésium, le solvant est évaporé sous pression réduite (20 mm de mercure; 2,7 kPa).

On obtient ainsi, avec un rendement de 90%, 0,567 g de méthyl-6 heptène-6 one-2 dont les caractéristiques sont les suivantes:

— P. $E._{101,3\,kPa}$ = 142—144° C
— Spectre infra-rouge à partir d'un film liquide: bandes caractéristiques à 3070, 1720, 1650 et 890 $cm^{-1}$
— Spectre de RMN ($CDCl_3$, $\delta$ en ppm, J en Hz): 1,65 (s, 3 H); 1,7 à 2,0 (mt, 4 H); 2,10 (s, 3 H); 2,30 (t, J=7, 3 H) et 4,65 (s, élargi, 2 H).

Les caractéristiques de ce produit sont en accord avec celles qui ont été décrites par Viola et coll., J. Amer. Chem. Soc., 89, 3462 (1967).

## Exemple 11

On opère dans les conditions de l'exemple 10 mais à partir de 0,126 g [$10^{-3}$ mole] de diméthyl-3,4 hexadiène-1,5 ol-3, de 0,120 g [$10^{-3}$ mole] de trifluoroacétate de lithium et de 0,120 g [0,28 $10^{-3}$ mole] de trifluoroacétate mercurique dans 7,5 $cm^3$ de chlorure de méthylène. Après 24 heures de réaction, on obtient, avec un rendement de 35%, 0,044 g d'octène-6 one-2 dont les caractéristiques sont les suivantes:

— Spectre infra-rouge à partir d'un film liquide: bandes caractéristiques à 3020, 1720 et 970 $cm^{-1}$
— Spectre RMN [$CDCl_3$, $\delta$ en ppm, J en Hz] 1,65 (dd, J=5 et 2, 3 H); 1,7 à 2,0 (mt, 4 H); 2,1 (s, 3 H); 2,35 (t,J=7, 2 H); 5,25—5,50 (mt, 2 H).

Exemple 12

On opère dans les conditions de l'exemple 10 mais à partir de 0,126 g [$10^{3-}$mole] de diméthyl-3,4 hexadiène-1,5 ol-3, de 0,160 g [$10^{-3}$ mole] de trifluorméthanesulfonate de lithium et de 0,100 g [0,23 $10^{-3}$ mole] de trifluoroacétate mercurique dans 10 $cm^3$ de chlorure de méthylène. Après 3 heures de réaction, on obtient avec un rendement de 52%, 0,065 g d'octène-6 one-2 dont les caractéristiques sont identiques à celles du produit décrit dans l'exemple 11.

Exemple 13

On maintient à une température voisine de 40°C, pendant 60 heures, un mélange de 0,195 g ($10^{-3}$ mole) d'isopropényl-4 diméthyl-3,7 octadièn-1,6 ol-3, de 0,121 g de trifluoracétate de lithium et de 0,091 g de trifluoracétate mercurique dans 10 $cm^3$ de chlorure de méthylène. On ajoute alors 0,002 g de borohydrure de sodium dans 0,1 $cm^3$ de soude 3 N. Le mélange réactionnel est extrait par 3 fois 10 $cm^3$ de chlorure de méthylène. Les extraits organiques sont séchés sur sulfate de magnésium. Après filtration et concentration à sec sous pression réduite (20 mm de mercure; 2,7 kPa), on obtient, avec un rendement de 40%, 0,078 g de diméthyl-6,10 undécadièn-6,9 one-2 dont les caractéristiques sont identiques à celles du produit de l'exemple 7.

Exemple 14

On maintient à une température voisine de 20°C, pendant 50 minutes, un mélange de 0,097 g ($5 \cdot 10^{-4}$ mole) d'isopropényl-4 diméthyl-3,7 octadien-1,6 ol-3, de 0,080 g de trifluorométhanesulfonate de lithium et de 0,044 g de trifluoracétate mercurique dans 10 $cm^3$ de chlorure de méthylène. On ajoute alors 0,001 g de borohydure de sodium en solution dans 0,05 $cm^3$ de soude 3 N. Le mélange réactionnel est extrait par 3 fois 10 $cm^3$ de chlorure de méthylène. Les extraits organiques sont séchées sur sulfate de magnésium. Après filtration et concentration à sec sous pression réduite (20 mm de mercure; 2,7 kPa), on obtient, avec un rendement de 60%, 0,058 g de diméthyl-6,10 undécadièn-6,9 one-2 dont les caractéristiques sont identiques à celles du produit de l'exemple 7.

Exemple 15

On maintient à une température voisine de 20°C, pendant 20 minutes, un mélange de 0,125 g ($5 \cdot 10^{-4}$ mole) de divinyl-1,2 cyclododécanol-1, de 0,80 g de trifluorométhanesulfonate de lithium et de 0,045 g de trifluoroacétate mercurique dans 5 $cm^3$ de chlorure de méthylène. Le mélange réactionnel est lavé par 3 fois 10 $cm^3$ d'une solution aqueuse saturée de bicarbonate de soude puis est extrait par 3 fois 10 $cm^3$ de chlorure de méthylène. Les extraits organiques sont séchées sur sulfate de magnésium. Après filtration et concentration à sec sous pression réduite (20 mm de mercure; 2,7 kPa), on obtient, avec un rendement de 69%, 0,085 g de cyclohexadecen-5 one 1.

**Revendications**

1. Procédé de préparation de composés carbonylés δ-éthyléniques de formule générale:

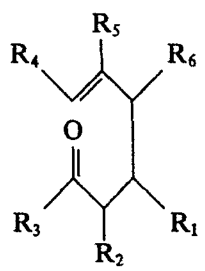

dans laquelle $R_1$, $R_2$, $R_4$, $R_5$ et $R_6$, identiques ou différents, représentent un atome d'hydrogène ou un radical acyclique contenant 1 à 20 atomes de carbone et dont la chaîne peut contenir une ou plusieurs doubles ou triples liaisons, $R_3$ représente un radical acyclique contenant 1 à 20 atomes de carbone et dont la chaîne peut contenir une ou plusieurs doubles ou triples liaisons, étant entendu que $R_1$ et $R_3$ peuvent former ensemble un radical triméthylène ou bien que $R_3$ et $R_4$ peuvent représenter ensemble un radical alcoylène $-(-CH_2)_n-$ dont un ou plusieurs atomes de carbone peuvent être substitués par un ou plusieurs radicaux alcoyles contenant 1 à 4 atomes de carbone et dans lequel n représente un nombre entier compris entre 3 et 20 inclusivement, par transposition d'un alcool diéthylénique de

formule générale:

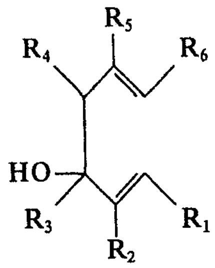

dans laquelle $R_1$, $R_2$, $R_3$, $R_4$, $R_5$ et $R_6$ sont définis comme précédemment, caractérisé en ce que l'on effectue la transposition à une température comprise entre —40° C et 80° C en présence d'une quantité pratiquement stoechiométrique d'un sel mercurique ou d'une quantité catalytique d'un sel mercurique et d'une quantité stoechiométrique d'un sel de lithium, et isole le produit obtenu éventuellement après traitement du milange réactionnel par un agent réducteur en milieu basique.

2. Procédé selon la revendication 1 caractérisé en ce que la transposition est effectuée à une température comprise entre — 5 et 30° C.

3. Procédé selon la revendication 1 caractérisé en ce que l'on opère avec une quantité pratiquement stoechiométrique d'un sel mercurique choisi parmi le chlorure, l'acétate ou le trifluoroacétate.

4. Procédé selon la revendication 1 caractérisé en ce que l'on opère avec une quantité catalytique de sel mercurique en présence d'une quantité stoechimétrique d'un sel de lithium choisi parmi le trifluoracétate et le trifluorométhane sulfonate.

5. Procédé selon la revendication 4 caractérisé en ce que le sel mercurique est le trifluoroacétate.

6. Procédé selon la revendication 4 caractérisé en ce que le sel mercurique est utilisé à raison de 0,01 à 0,3 mole par mole d'alcool diéthylénique mis en oeuvre.

7. Procédé selon la revendication 1 caractérisé en ce que l'agent réducteur éventuellement utilisé pour traiter le mélange réactionnel est un borohydrure alcalin tel que le borohydrure de sodium.

8. Procédé selon la revendication 1 caractérisé en ce que, lorsque l'on utilise une quantité catalytique d'un sel mercurique et une quantité stoechiométrique d'un sel de lithium on isole le produit obtenu après traitement du mélange réactionnel par une solution aqueuse basique.

9. Procédé selon la revendication 1 caractérisé en ce que l'on met en oeuvre un alcool diéthylénique de formule générale:

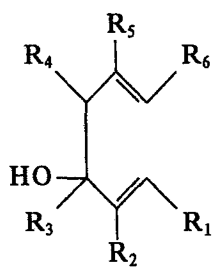

dans laquelle $R_2$ et $R_6$ représentent un atome d'hydrogène, $R_1$ et $R_5$ représentent un atome d'hydrogène ou un radical alcoyle contenant 1 à 4 atomes de carbone, $R_3$ représente un radical alcoyle contenant 1 à 4 atomes de carbone, $R_4$ représente un atome d'hydrogène ou un radical alcoyle contenant 1 à 4 atomes de carbone ou un radical alcényle contenant 2 à 4 atomes de carbone, ou bien, $R_1$ et $R_3$ représentent ensemble un radical triméthylène, les symboles $R_2$, $R_4$, $R_5$ et $R_6$ étant définis comme ci-dessus, ou bien $R_3$ et $R_4$ représentent ensemble un radical alcoylène $-(CH_2)_n-$ dans laquel n est compris entre 3 et 12 inclusivement, les symboles $R_1$, $R_2$, $R_5$ et $R_6$ étant définis comme ci-dessus.

**Patentansprüche:**

1. Verfahren zur Herstellung von $\delta$-äthylenischen Carbonylverbindungen der allgemeinen Formel

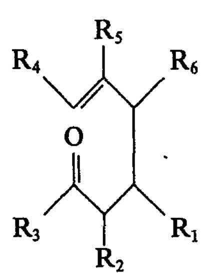

in welcher $R_1$, $R_2$, $R_4$, $R_5$ und $R_6$, die gleich oder voneinander verschieden sind, ein Wasserstoffatom oder einen acyclischen Rest, der 1 bis 20 Kohlenwasserstoffatome enthält und deren Kette eine oder mehrere Doppel- oder Dreifachbindungen enthalten kann, $R_3$ einen acyclischen Rest mit 1 bis 20 Kohlenstoffatomen darstellt, dessen Kette eine oder mehrere Doppel- oder Dreifachbindungen enthalten kann, wobei $R_1$ und $R_3$ gemeinsam einen Trimethylenrest bilden können oder $R_3$ und $R_4$ gemeinsam einen $-(-CH_2)_n$-Rest darstellen können, von denen ein oder mehrere Kohlenstoffatome durch einen oder mehrere Alkylreste mit 1 bis 4 Kohlenstoffatomen substituiert sein können, und in welcher n eine ganze Zahl zwischen 3 und 20 darstellt, durch Umlagerung eines diäthylenischen Alkohols der allgemeinen Formel

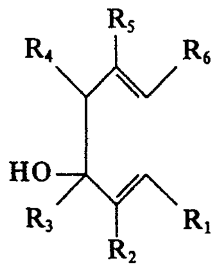

in welcher $R_1$, $R_2$, $R_3$, $R_4$, $R_5$ und $R_6$ die obige Bedeutung haben, dadurch gekennzeichnet, daß man die Umlagerung bei einer Temperatur zwischen —40° C und 80° C in Gegenwart einer praktisch stöchiometrischen Menge eines Quecksilber(II)salzes oder einer katalytischen Menge eines Quecksilber(II)salzes und einer stöchiometrischen Menge eines Lithiumsalzes ausführt und das erhaltene Produkt, gegebenenfalls nach Behandlung der Reaktionsmischung mit einem Reduktionsmittel in basischem Milieu, isoliert.

2. Verfahren nach Anspruch 1, dadurch gekennzeichnet, daß die Umlagerung bei einer Temperatur zwischen — 5 und 30° C durchgeführt wird.

3. Verfahren nach Anspruch 1, dadurch gekennzeichnet, daß man mit einer praktisch stöchiometrischen Menge eines Quecksilber(II)salzes, ausgewählt aus dem Chlorid, dem Acetat bzw. dem Trifluoracetat, arbeitet.

4. Verfahren nach Anspruch 1, dadurch gekennzeichnet, daß man mit einer katalytischen Menge des Quecksilber(II)salzes in Gegenwart einer praktisch stöchiometrischen Menge eines Lithiumsalzes, ausgewählt aus dem Trifluoracetat und dem Trifluormethansulfonat, arbeitet.

5. Verfahren nach Anspruch 4, dadurch gekennzeichnet, daß das Quecksilber(II)salz das Trifluoracetat ist.

6. Verfahren nach Anspruch 4, dadurch gekennzeichnet, daß das Quecksilber(II)salz in einer Menge von 0,01 Mol bis 0,3 Mol pro Mol des eingesetzten diäthylenischen Alkohols verwendet wird.

7. Verfahren nach Anspruch 1, dadurch gekennzeichnet, daß das gegebenenfalls zur Behandlung der Reaktionsmischung verwendete Reduktionsmittel ein Alkaliborhydrid, z. B. Natriumborhydrid, ist.

8. Verfahren nach Anspruch 1, dadurch gekennzeichnet, daß das bei Verwendung einer katalytischen Menge eines Quecksilber(II)salzes und einer stöchiometrischen Menge eines Lithiumsalzes erhaltene Produkt nach Behandlung der Reaktionsmischung mit einer basischen wäßrigen Lösung isoliert wird.

9. Verfahren nach Anspruch 1, dadurch gekennzeichnet, daß man einen diäthylenischen Alkohol der allgemeinen Formel

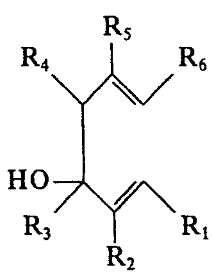

einsetzt, in welcher $R_2$ und $R_6$ ein Wasserstoffatom darstellen, $R_1$ und $R_5$ ein Wasserstoffatom oder einen Alkylrest mit 1 bis 4 Kohlenstoffatomen darstellen, $R_3$ einen Alkylrest mit 1 bis 4 Kohlenstoffatomen bedeutet, $R_4$ ein Wasserstoffatom oder einen Alkylrest mit 1 bis 4 Kohlenstoffatom oder einen Alkylenrest mit 2 bis 4 Kohlenstoffatomen darstellen oder $R_1$ und $R_3$ zusammen eine Trimethylengruppe darstellen, wobei die Symbole $R_2$, $R_4$, $R_5$ und $R_6$ die obige Bedeutung haben, oder $R_3$ und $R_4$ zusammen einen Alkylenrest $-(CH_2)_n-$ darstellen, worin n für 3 bis 12 steht, wobei die Symbole $R_1$, $R_2$, $R_5$ und $R_6$ die obige Bedeutung haben.

**Claims**

1. Process for the preparation of δ-ethylenic carbonyl compounds of the general formula:

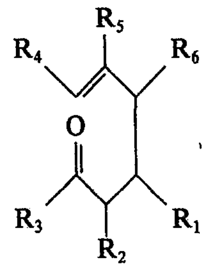

in which $R_1$, $R_2$, $R_4$, $R_5$ and $R_6$, which are identical or different, represent a hydrogen atom or an acyclic radical which contains 1 to 20 carbon atoms and the chain of which can contain one or more double or triple bonds, and $R_3$ represents an acyclic radical which contains 1 to 20 carbon atoms and the chain of which can contain one or more double or triple bonds, it being understood that $R_1$ and $R_3$ can together form a trimethylene radical, or alternatively that $R_3$ and $R_4$ can together represent an alkylene radical $-(-CH_2)_n-$, one or more carbon atoms of which can be substituted by one more alkyl radicals containing 1 to 4 carbon atoms, and in which n represents an integer between 3 and 20 inclusive, by the rearrangement of a diethylenic alcohol of the general formula:

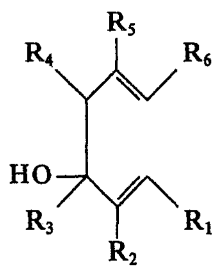

in which $R_1$, $R_2$, $R_3$, $R_4$, $R_5$ and $R_6$ are defined as above, characterised in that the rearrangement is carried out at a temperature of between −40° C and 80° C in the presence of a vartually stoichiometric amount of a mercuric salt, or in the presence of a catalytic amount of a mercuric salt and a stoichiometric amount of a lithium salt, and the product obtained is isolated, if appropriate after treatment of the reaction mixture with a reducing agent in a basic medium.

2. Process according to Claim 1, characterised in that the rearrangement is carried out at a temperature of between −5 and 30° C.

3. Process according to Claim 1, characterised in that the reaction is carried out with a virtually stoichiometric amount of a mercuric salt chosen from amongst the chloride, the acetate or the trifluoroacetate.

4. Process according to Claim 1, characterised in that the reaction is carried out with a catalytic amount of mercuric salt in the presence of a stoichiometric amount of a lithium salt chosen from amongst the trifluoroacetate and the trifluoromethanesulphonate.

5. Process according to Claim 4, characterised in that the mercuric salt is the trifluoroacetate.

6. Process according to Claim 4, characterised in that the mercuric salt is used in an amount of 0,01 to 0,3 mol per mol of diethylenic alcohol employed.

7. Process according to Claim 1, characterised in that the reducing agent which may be used to treat the reaction mixture is an alkali metal borohydride such as sodium borohydride.

8. Process according to Claim 1, characterised in that, when using a catalytic amount of a mercuric salt and a stoichiometric amount of a lithium salt, the product obtained is isolated after treatment of the reaction mixture with a basic aqueous solution.

9. Process according to Claim 1, characterised in that a diethylenic alcohol of the general formula:

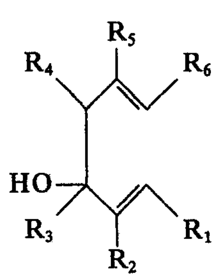

is used, in which $R_2$ and $R_6$ represent a hydrogen atom, $R_1$ and $R_5$ represent a hydrogen atom or an alkyl radical containing 1 to 4 carbon atoms, $R_3$ represents an alkyl radical containing 1 to 4 carbon atoms and $R_4$ represents a hydrogen atom, an alkyl radical containing 1 to 4 carbon atoms or an alkenyl radical containing 2 to 4 carbon atoms, or alternatively $R_1$ and $R_3$ together represent a trimethylene radical, the symbols $R_2$, $R_4$, $R_5$ and $R_6$ being defined as above, or alternatively $R_3$ and $R_4$ together represent an alkylene radical $-(CH_2)_n-$, in which n is between 3 and 12 inclusive, the symbols $R_1$, $R_2$, $R_5$ and $R_6$ being defined as above.